# EUROPEAN PATENT SPECIFICATION

(11) **EP 4 074 301 B1**
(45) Date of publication and mention of the grant of the patent: **28.01.2026**
(21) Application number: 20898792.5
(22) Date of filing: 11.12.2020
(51) Int. Cl.: A61K 8/33, A61K 8/36, A61K 8/39, A61K 8/73, A61Q 5/02, A61K 8/41, A61K 8/46, A61K 8/86, A61K 8/04, A61K 8/81

(54) **HAIR CLEANSER COMPOSITION, KIT AND METHOD FOR HAIR CLEANING**
HAARREINIGUNGSZUSAMMENSETZUNG, KIT UND VERFAHREN ZUR HAARREINIGUNG
COMPOSITION DE NETTOYANT CAPILLAIRE, KIT ET PROCÉDÉ POUR NETTOYER LES CHEVEUX

(30) Priority: 12.12.2019 TW 108145578; 08.12.2020 TW 109143205
(43) Date of publication of application: 19.10.2022
(73) Proprietor: Kao Corporation, Chuo-ku Tokyo 103-8210 (JP)
(72) Inventor: CHAN, Yungchen, Hsinchu Hsien Taiwan 30352 (TW); WANG, Shufen, Hsinchu Hsien Taiwan 30352 (TW); FUKUHARA, Chikako, Tokyo 131--0044 (JP)
(74) Representative: Hoffmann Eitle
(86) International application number: PCT/IB2020/061796
(87) International publication number: WO 2021/116986

(56) References cited:
- EP-A1- 2 191 814
- JP-A- 2012 001 512
- JP-A- 2013 155 143
- JP-A- 2014 037 403
- JP-A- 2014 040 569
- JP-A- 2014 131 988
- JP-A- 2014 508 789
- JP-A- 2015 168 666
- JP-A- 2017 066 114
- JP-A- 2017 066 114
- JP-A- 2018 177 652
- JP-A- 2018 177 652
- US-A1- 2014 079 658

## Description

### Field of the Invention

The present invention is defined in the appended claims and relates to a hair cleansing composition, a kit, and a hair cleansing method using the hair cleansing composition.

### Description of the Prior Art

By making the hair cleansing composition into a foam and applying it to the hair, the hair cleansing composition can be easily dispersed over the entire hair and thus easily spread on hair during application. Therefore, in recent years, there is a need to develop a hair cleansing agent for filling a foam discharge device.

For example, Patent Document 1 discloses a hair cleansing composition for filling a foam discharge device and comprising a particular amide-amine type amphoteric surfactant and a particular cationic cellulose.

Patent Document 2 discloses a hair cleansing composition for filling a foam discharge device and comprising an anionic surfactant, a cationic polymer and a polyoxypropylene polyglyceryl ether. See also US 2014/079658 A1 (TERAZAKI HIROYUKI [JP] ET AL) 20 March 2014 (2014-03-20).

### Prior Art Documents: Patent Documents

Patent Document 1: Japanese Patent Publication No. 2013-155143
Patent Document 2: Japanese Patent Publication No. 2018-177652

### Summary of the Invention

The present invention relates to the following aspects.

A hair cleansing composition comprises component (A), component (B), component (C), and one or more components selected from the group consisting of component (D-1) and component (D-2), as defined in the claims.

Another aspect of the present invention provides a hair cleansing kit, which comprises a foam discharge device and the hair cleansing composition as identified above and in the appended claims.

In addition, the present invention provides a hair cleansing method, which comprises applying the hair cleansing composition,as identified above and in the appended claims, to the hair for cleansing, followed by scrubbing or rinsing.

Although the compositions disclosed in Patent Document 1 and Patent Document 2 can be applied to a foam discharge device, the viscosity and performance of these compositions at low temperatures (especially as low as 5°C) still need to be improved. For example, the compositions shown in Patent Document 1 and Patent Document 2 show good storage stability at low temperatures; however, in some composition embodiments, the problems occur that the compositions have difficulty being discharged from a foam discharge device due to too high of a viscosity at low temperatures, and the foam quality at low temperatures also needs to be improved.

As a result of studies conducted by the present inventors, it is found that by adding a dialkyl ether and one or more compounds selected from the group consisting of a compound represented by the general formula (5) and a polyoxypropylene polyglyceryl ether to a hair cleansing composition comprising an anionic surfactant and a cationic polymer, the above-mentioned problems are solved, and sufficient smoothness can be provided to the hair during scrubbing or rinsing:

R^{a}-O-(AlkO)ₘ-R^{b} (5)

where in the general formula (5), R^{a} represents a linear or branched alkyl or alkenyl group having a carbon number from 8 or more to 10 or less, AlkO represents a linear or branched oxyalkylene group having a carbon number from 2 or more to 4 or less, m is a number of oxyalkylene groups and represents an average from 0.5 or more to 3.5 or less, and R^{b} represents a hydrogen atom or methyl.

The hair cleansing composition according to the present invention not only can maintain a relatively low viscosity at low temperatures (e.g., 5° C.), but also can improve the foam discharge performance at low temperatures and increase the convenience of operation in a low temperature environment. In addition, the hair cleansing composition of the present invention also has good foam quantity and foam quality at low temperatures and provides sufficient smoothness to the hair during scrubbing or rinsing.

### Detailed Description

The present invention will be described in detail hereinafter.

The hair cleansing composition as defined in claim 1.

### <Component (A)>

Component (A) is a surfactant comprising at least one anionic surfactant. In addition to the anionic surfactant, the surfactants that can be added to Component (A) include, for example, a cationic surfactant, a nonionic surfactant, and an amphoteric surfactant, etc. Here, the anionic surfactant can be used alone or in combination with one or more of the surfactants exemplified above.

From the perspective of improving the foam quality of the composition during low-temperature discharge, imparting good foamability (foam amount) when rinsing hair, and providing smoothness to hair during scrubbing or rinsing, the total content of component (A) may be, for example, 5 mass% or more, preferably 6 mass% or more, more preferably 7 mass% or more, further preferably 8 mass% or more, still further preferably 9 mass% or more, and most preferably 11 mass% or more, relative to the total hair cleansing composition. Furthermore, from the same perspective, the total content of component (A) may be, for example, 25 mass% or less, preferably 22 mass% or less, more preferably 20 mass% or less, further preferably 19 mass% or less, still further preferably 17 mass% or less, and still further preferably 15 mass% or less.

More particularly, from the above-mentioned perspective, the total content of component (A) may be, for example, from 5 mass% or more to 25 mass% or less, preferably from 6 mass% or more to 22 mass% or less, more preferably from 7 mass% or more to 20 mass% or less, further preferably from 8 mass% or more to 19 mass% or less, still further preferably from 9 mass% or more to 17 mass% or less, and still further preferably from 11 mass% or more to 15 mass% or less.

### <Anionic surfactant>

The anionic surfactant may be any anionic surfactant as long as it is suitable for use in the hair cleansing composition. Embodiments of the anionic surfactant include: a sulfate-type anionic surfactant, such as an alkyl sulfate, an alkenyl sulfate, a polyoxyalkylene alkyl ether sulfate, and a polyoxyalkylene alkenyl ether sulfate; a sulfonic acid-type anionic surfactant, such as an alkyl sulfosuccinate, an alkyl polyoxyalkylene sulfosuccinate, and an alkyl sulfonate; and a carboxylic acid-type anionic surfactant, such as a higher fatty acid salt, and a polyoxyalkylene alkyl ether carboxylic acid or a salt thereof. The above surfactants may be used alone or in combination of two or more.

The anionic surfactant is preferably a polyoxyalkylene alkyl ether sulfate, a polyoxyalkylene alkenyl ether sulfate, an alkyl sulfate, or an alkenyl sulfate, where the polyoxyalkylene alkyl ether sulfate is preferably a polyoxyethylene alkyl ether sulfate represented by the general formula (1) below:

R¹O(CH₂CH₂O)ₐSO₃M (1)

[In the general formula (1), R¹ represents a linear or branched alkyl or alkenyl group having a carbon number from 8 or more to 18 or less, M represents an alkali metal, alkaline earth metal, ammonium, alkanolamine or basic amino acid, and a is an average molar number of addition of ethylene oxide and is a number from 0.5 or more to 5 or less on average].

In these polyoxyethylene alkyl ether sulfates, from the perspective of maintaining foamability and good foam feel, R¹ in the general formula (1) is preferably a linear alkyl group having a carbon number from 12 or more to 14 or less. The average molar number a of addition of ethylene oxide (EO) is preferably from 0.9 or more to 4 or less, and more preferably from 1 or more to 3 or less. The polyoxyethylene alkyl ether sulfate is preferably a polyoxyethylene alkyl ether sulfate where M is ammonium or sodium, and more preferably ammonium laureth sulfate and sodium laureth sulfate.

In addition, when the anionic surfactant includes a carboxylic acid-type anionic surfactant, the carboxylic acid-type anionic surfactant is preferably a polyoxyalkylene alkyl ether carboxylate, and more preferably a polyoxyethylene alkyl ether carboxylate represented by the general formula (2) below:

R³O-(CH₂-CH₂O)ₛ-CH₂COOX (2)

[In the general formula (2), R³ represents a linear or branched alkyl or alkenyl group having a carbon number from 10 or more to 18 or less, X represents a cation from an alkali metal, alkaline earth metal, ammonium, alkanolamine or basic amino acid, and s is an average molar number of addition of ethylene oxide and is a number from 0.5 or more to 15 or less on average].

In these carboxylic acid-type anionic surfactants, from the perspective of creating abundant foam when rinsing, R³ in the general formula (2) is preferably a linear alkyl group having a carbon number from 12 or more to 16 or less. The average molar number s of addition of ethylene oxide (EO) is preferably from 1 or more to 6 or less. The carboxylic acid-type anionic surfactant is preferably a polyoxyethylene alkyl ether carboxylate where X is ammonium or sodium, and more preferably ammonium laureth carboxylate and sodium laureth carboxylate.

In addition, the anionic surfactant further preferably includes a polyoxyalkylene alkyl ether carboxylate, and a polyoxyethylene alkyl ether sulfate.

From the perspective of improving foamability, the content of the anionic surfactant in the hair cleansing composition is, for example, 5 mass% or more, preferably 6 mass% or more, more preferably 7 mass% or more, and further preferably 8 mass% or more relative to the total hair cleansing composition. Furthermore, from the perspective of improving foam quality and imparting smoothness to hair during scrubbing or rinsing, the content of the anionic surfactant is, for example, 25 mass% or less, preferably 22 mass% or less, more preferably 20 mass% or less, further preferably 19 mass% or less, still further preferably 17 mass% or less, still further preferably 15 mass% or less, still further preferably 13 mass% or less, and still further preferably 12 mass% or less relative to the total hair cleansing composition.

More particularly, from the above-mentioned perspective, the content of the anionic surfactant is, for example, from 5 mass% or more to 25 mass% or less, preferably from 6 mass% or more to 22 mass% or less, more preferably from 7 mass% or more to 20 mass% or less, further preferably from 8 mass% or more to 19 mass% or less, still further preferably from 8 mass% or more to 17 mass% or less, still further preferably from 8 mass% or more to 15 mass% or less, still further preferably from 8 mass% or more to 13 mass% or less, and still further preferably from 8 mass% or more to 12 mass% or less.

Moreover, from the perspective of improving the cleansing effect, the mass ratio of the content of the anionic surfactant to the content of component (A) in the hair cleansing composition ((anionic surfactant)/(A)) is preferably 0.25 or more, more preferably 0.3 or more, and further preferably 0.5 or more. From the same perspective, the mass ratio of the content of the anionic surfactant to the content of component (A) in the hair cleansing composition is preferably 1 or less, more preferably 0.9 or less, and further preferably 0.8 or less.

More particularly, from the above-mentioned perspective, the mass ratio of the content of the anionic surfactant to the content of component (A) in the hair cleansing composition may be, for example, from 0.25 or more to 1 or less, preferably from 0.3 or more to 0.9 or less, and more preferably from 0.5 or more to 0.8 or less.

### <Cationic surfactant>

A cationic surfactant may be optionally used in the hair cleansing composition of the present invention. Embodiments of the cationic surfactant include, for example, a tertiary amine compound or a salt thereof, a quaternary ammonium salt, and the like. More particularly, it can be the cationic surfactants described in Japanese Patent Publication No. 2017-019748. The cationic surfactants may be used alone or in combination of two or more.

If a cationic surfactant is added, from the perspective of imparting smoothness to hair during scrubbing or rinsing, the content of the cationic surfactant in the hair cleansing composition can be, for example, 0.05 mass% or more, preferably 0.1 mass% or more, more preferably 0.2 mass% or more, and further preferably 0.3 mass% or more relative to the total hair cleansing composition. From the same perspective, the content of the cationic surfactant is preferably 2 mass% or less, more preferably 1 mass% or less, and further preferably 0.7 mass% or less.

More particularly, from the above-mentioned perspective, the content of the cationic surfactant is, for example, from 0.05 mass% or more to 2 mass% or less, preferably from 0.1 mass% or more to 1 mass% or less, more preferably from 0.2 mass% or more to 0.7 mass% or less, and further preferably from 0.3 mass% or more to 0.7 mass% or less.

### <Nonionic surfactant>

Component (A) may further comprise a nonionic surfactant if necessary. The addition of a non-ionic surfactant is beneficial to improve the cleansing effect.

The nonionic surfactant may be any nonionic surfactant as long as it is suitable for use in the hair cleansing composition. Embodiments of the nonionic surfactant include: an alkyl polyglycoside; a polyoxyalkylene alkyl ether, such as a polyoxyethylene lauryl ether, a polyoxyethylene stearyl ether, and a polyoxyethylene isostearyl ether; ethoxylated hydrogenated castor oil; a glycerol fatty acid ester; a sorbitan fatty acid ester; a polyoxyalkylene sorbitan fatty acid ester; a (poly)ethylene glycol fatty acid ester, such as polyethylene glycol fatty acid ester; a polyoxyalkylene fatty acid ester; a polyoxyalkylene sorbitan fatty acid ester, such as polyoxyethylene sorbitan fatty acid ester; a monoalkyl glyceryl ether, such as 2-ethylhexyl glyceryl ether or isodecyl glyceryl ether; a monoalkenyl glyceryl ether; and a fatty acid monoalkanolamide, such as coconut oil fatty acid monoethanolamide, and coconut oil fatty acid N-methyl monoethanolamide. The alkyl group in the nonionic surfactant may be linear or branched. The nonionic surfactants may be used alone or in combination of two or more.

From the perspective of obtaining good foamability, a nonionic surfactant comprising a compound represented by the general formula (3) is preferable:

R²-O-(CH₂CH₂-O)ₙ-H (3).

[In the general formula (3), R² represents a linear or branched alkyl or alkenyl group having a carbon number from 12 to 24, and n represents a number from 1 to 50.]

R² in the general formula (3) is preferably a linear alkyl group having a carbon number from 12 to 20. n is preferably a number from 1 or more to 25 or less.

When a nonionic surfactant is added, from the perspective of improving the foam quality of the composition when it is discharged at a low temperature (5°C), imparting good foamability (foam amount) when rinsing hair, and imparting smoothness to hair during scrubbing or rinsing, the content of the nonionic surfactant in the hair cleansing composition may be, for example, 0.1 mass% or more, preferably 0.5 mass% or more, and more preferably 1 mass% or more relative to the total hair cleansing composition. From the same perspective, the content of the nonionic surfactant t is preferably 10 mass% or less, more preferably 7 mass% or less, and further preferably 5 mass% or less.

More particularly, from the above-mentioned perspective, the content of the cationic surfactant may be, for example, from 0.1 mass% or more to 10 mass% or less, preferably from 0.5 mass% or more to 7 mass% or less, and more preferably from 1 mass% or more to 5 mass% or less.

### <Amphoteric surfactant>

The amphoteric surfactant can be any amphoteric surfactant as long as it is suitable for use in the hair cleansing composition. Embodiments of the amphoteric surfactant include a betaine-type surfactant, an amino acid-type surfactant, an imidazoline-type surfactant, and an amine oxide-type surfactant. The amphoteric surfactants may be used alone or in combination of two or more.

Embodiments of betaine-type surfactants include, for example, a carbonylbetaine-type surfactant, such as an alkylcarbonylbetaine; a sulfobetaine-type surfactant, such as an alkylsulfobetaine, an alkylhydroxysulfobetaine (for example, lauryl hydroxysultaine), and an alkylamidohydroxysulfobetaine; an alkylamido amine-type betaine; and an alkylimidazoline-type betaine.

From the perspective of foam quality, and feeling during scrubbing or rinsing, the amphoteric surfactant is preferably an alkyl amidopropyl betaine or an alkyl hydroxysulfobetaine having an alkyl group with 8 to 18 carbon atoms, more preferably alkyl hydroxysulfobetaine, for example, lauryl hydroxysultaine or myristyl hydroxy sulfobetaine, and further preferably lauryl hydroxysultaine.

The alkyl amidopropyl betaine having an alkyl group with 8 to 14 carbon atoms is preferably laurylamidopropryl betaine.

From the perspective of obtaining good foamability and imparting smoothness to hair during scrubbing or rinsing, the content of the amphoteric surfactant in the hair cleansing composition is preferably 0.1 mass% or more, more preferably 0.2 mass% or more, and further preferably 0.5 mass% or more relative to the total hair cleansing composition. From the same perspective, the content of the amphoteric surfactant is preferably 10 mass% or less, more preferably 5 mass% or less, further preferably 4 mass% or less, still further preferably 3 mass% or less, and most preferably 2 mass% or less.

More particularly, from the above-mentioned perspective, the content of the amphoteric surfactant may be, for example, from 0.1 mass% or more to 10 mass% or less, preferably from 0.2 mass% or more to 5 mass% or less, more preferably from 0.5 mass% or more to 4 mass% or less, further preferably from 0.5 mass% or more to 3 mass% or less, and still further preferably from 0.5 mass% or more to 2 mass% or less.

### <Component (B)>

Component (B) is a cationic polymer. The cationic polymer is a polymer having a cationic group such as quaternary ammonium or a group capable of ionization into a cationic group (such as primary, secondary or tertiary amine group).

Embodiments of cationic polymers include cationic guar gum (e.g., Jaguar C14, Jaguar C17, or Jaguar Excel manufactured by Solvay Corporation), cationic tara gum (e.g., Catinal CTR-100 manufactured by TOHO Chemical Industry Co., Ltd.), cationic locust bean gum (locust bean hydroxypropyltrimonium chloride; e.g. Catinal CLB-100 manufactured by TOHO Chemical Industry Co., Ltd.), cationic fenugreek gum (e.g. Catinal CF-100 manufactured by TOHO Chemical Industry Co., Ltd.), cationic galactomannan, cationic xantham gum, cationic cellulose (e.g. PPG-2 hydroxypropyltrimethylammonium cellulose, and polyquaternium-10 (such as Poiz C-60H, Poiz C-80M, and Poiz C-150L manufactured by Kao Corporation)), cationic starch, a homopolymer of a diallyldimethyl quaternary ammonium salt (polyquaternium-6; e.g. Merquat 100 manufactured by Lubrizol Corporation), a diallyldimethyl quaternary ammonium salt/acrylamide copolymer (polyquaternium-7; e.g. Merquat 550, Merquat 740, and Merquat 2200 manufactured by Lubrizol Corporation), a diallyldimethyl quaternary ammonium salt/acrylic acid copolymer (polyquaternium-22; e.g. Merquat 280 manufactured by Lubrizol Corporation), a N,N-dimethylaminoethylmethacrylate diethylsulfate-N,N-dimethylacrylamide-polyethylene glycol dimethacrylate copolymer (polyquaternium-52; e.g. Sofcare KG-101E and Sofcare KG-301W manufactured by Kao Corporation), a quaternized polyvinylpyrrolidone derivative, a polyethylene glycol-polyamine condensate, a vinylimidazolium trichloride/vinylpyrrolidone copolymer, a hydroxyethylcellulose/dimethyldiallylammonium chloride copolymer (polyquaternium-4; e.g. Cellcoat L200 manufactured by AkzoNobel), a vinylpyrrolidone/quaternized dimethylaminoethyl methacrylate copolymer (polyquaternium-11; e.g. Gafquat 755 manufactured by Ashland Inc.), a polyvinylpyrrolidone/aminoalkyl acrylate copolymer, a polyvinylpyrrolidone/aminoalkyl acrylate/vinyl caprolactam copolymer (e.g. copolymers 845, 937, and 958 manufactured by Ashland Inc.), vinylpyrrolidone/methacrylamidopropyltrimethylammonium chloride copolymer (Gafquat HS-100 manufactured by Ashland Inc.), an alkyl acrylamide/acrylate/alkylaminoalkyl acrylamide/polyethylene glycol methacrylate copolymer, adipic acid/dimethylaminohydroxypropyl ethylene triamine copolymer (Cartaretin manufactured by Sandoz Inc., USA), cationic polymers described in Japanese Patent Publication No. 53-139734 and Japanese Patent Publication No. 60-36407, and others.

From the perspective of imparting good foamability (foam amount) and good form quality when rinsing hair, and imparting smoothness to hair during scrubbing or rinsing, component (B) is preferably one or more cationic polymers selected from the group consisting of cationic guar gum, PPG-2 hydroxypropyltrimethylammonium cellulose, cationic galactomannan, cationic starch, polyquaternium-6, polyquaternium-7, polyquaternium-10, polyquaternium-22, and polyquaternium-52. Component (B) is further preferably at least one cationic polymer selected from the group consisting of cationic guar gum, PPG-2 hydroxypropyltrimethylammonium cellulose, cationic galactomannan, polyquaternium-6, polyquaternium-7, polyquaternium-10, and polyquaternium-22. Components (B) may be used alone or in combination of two or more.

From the perspective of imparting good foam quality when rinsing hair and imparting smoothness to hair during scrubbing or rinsing, the content of component (B) in the hair cleansing composition is preferably 0.01 mass% or more, more preferably 0.015 mass% or more, and further preferably 0.03 mass% or more relative to the total hair cleansing composition. Furthermore, from the perspective of improving the foam quality during discharge, the content of component (B) is preferably 1.0 mass% or less, more preferably 0.8 mass% or less, further preferably 0.7 mass% or less, still further preferably 0.5 mass% or less, still further preferably 0.30 mass% or less, and still further preferably 0.25 mass% or less.

More particularly, from the above-mentioned perspective, the total content of component (B) may be, for example, from 0.01 mass% or more to 1.0 mass% or less, preferably from 0.015 mass% or more to 0.8 mass% or less, more preferably from 0.03 mass% or more to 0.7 mass% or less, further preferably from 0.03 mass% or more to 0.5 mass% or less, still further preferably from 0.03 mass% or more to 0.30 mass% or less, and still further preferably from 0.03 mass% or more to 0.25 mass% or less.

### <Component (C)>

Component (C) is a dialkyl ether. The present inventors find that by adding a dialkyl ether (hereinafter, also referred to as component (C)) in the hair cleansing composition, it is effective to prevent the viscosity of the hair cleansing composition from being greatly increased at low temperatures, so that the foam can be easily discharged from a foam discharge device, which greatly improves convenience of operation. In addition, good foam quality can be obtained at low temperatures, and sufficient smoothness can be imparted to hair when scrubbing or rinsing.

Component (C) is represented by the following general formula (4):

R⁴-O-R⁵ (4)

In the above general formula (4), R⁴ and R⁵ are independently an alkyl group having a carbon number from 5 or more to 14 or less, and more preferably an alkyl group having a carbon number from 6 or more to 10 or less.

From the perspective of further reducing the viscosity of the hair cleansing composition and improving the foam quality during discharge at low temperatures, the content of component (C) in the hair cleansing composition is preferably 0.01 mass% or more, more preferably 0.05 mass% or more, and further preferably 0.1 mass% or more relative to the total hair cleansing composition. From the same perspective, the content of component (C) is preferably 5 mass% or less, more preferably 4 mass% or less, further preferably 3 mass% or less, still further preferably 2.0 mass% or less, and still further preferably 1 mass% or less.

More particularly, from the above-mentioned perspective, the content of component (C) is, for example, from 0.01 mass% or more to 5 mass% or less, preferably from 0.05 mass% or more to 4 mass% or less, more preferably from 0.1 mass% or more to 3 mass% or less, further preferably from 0.1 mass% or more to 2.0 mass% or less, and still further preferably from 0.1 mass% or more to 1 mass% or less.

From the perspective of improving the foam quality of the composition during low-temperature discharge, imparting good foamability (foam amount) when rinsing hair, and providing smoothness to hair during scrubbing or rinsing, the mass ratio ((C)/(B)) of the content of component (C) to the content of component (B) in the hair cleansing composition is 2 or more, preferably 3 or more, and more preferably 5 or more. From the same perspective, the mass ratio ((C)/(B)) is 50 or less, more preferably 30 or less, and further preferably 25 or less.

More particularly, from the above-mentioned perspective, the mass ratio ((C)/(B)) is from 2 or more to 50 or less, preferably from 3 or more to 30 or less, and more preferably from 5 or more to 25 or less.

### <Component (D-1)>

Component (D-1) is a compound represented by the general formula (5) below:

R^{a}-O-(AlkO)ₘ-R^{b} (5)

In the general formula (5), R^{a} represents a linear or branched alkyl or alkenyl group having a carbon number of 8 or more to 10 or less, and AlkO represents a linear or branched oxyalkylene group having a carbon number of 2 or more to 4 or less (for example, -CH₂CH₂O-, -CH₂CH₂CH₂O-, -CH₂CH₂CH₂CH₂O- or -CH₂CH(CH₃)O-), m is a number of oxyalkylene group and represents an average of 0.5 or more to 3.5 or less, and R^{b} represents a hydrogen atom or methyl.

Examples of component (D-1) include, for example, PPG-3 octyl ether. From the perspective of imparting smoothness to hair during scrubbing or rinsing, the content of component (D-1) is preferably 0.05 mass% or more, and more preferably 0.1 mass% or more, relative to the total hair cleansing composition. From the same perspective, the content of component (D-1) is preferably 3 mass% or less, and more preferably 1.5 mass% or less.

More particularly, from the above-mentioned perspective, the content of component (D-1) may be, for example, from 0.05 mass% or more to 3 mass% or less, and preferably from 0.1 mass% or more to 1.5 mass% or less.

### <Component D-2>

Component (D-2) is a polyoxypropylene polyglyceryl ether, which is a condensate of 2 or more molecules of glycerol with 2 or more molecules of propylene oxide (or propylene glycol), and further a condensate of polyglycerol condensed from 2 or more molecules of glycerol with 2 or more molecules of propylene oxide (or propylene glycol).

From the perspective of reducing the viscosity of the hair cleansing composition, obtaining good foam quality when rinsing hair, and imparting smoothness to hair during scrubbing or rinsing, the degree of polymerization of glycerol in component (D-2) is 2 or more, preferably from 2 or more to 10 or less, more preferably from 2 or more to 5 or less, further preferably from 2 or more to 3 or less, and further preferably 2.

Furthermore, from the perspective of imparting good foam quality when rinsing hair and imparting smoothness to hair during scrubbing or rinsing, the molar number of addition of propylene oxide in component (D-2) is preferably 3 or more, more preferably 5 or more, further preferably 7 or more, and still further preferably 9 or more. Furthermore, it is preferably 24 or less, more preferably 20 or less, further preferably 15 or less, and still further preferably 14 or less.

More particularly, from the above-mentioned perspective, the molar number of addition of propylene oxide may be, for example, from 3 or more to 24 or less, preferably from 5 or more to 20 or less, more preferably from 7 or more to 15 or less, and further preferably from 9 or more to 14 or less.

When propylene glycol is used, the molar number of addition of propylene glycol in component (D-2) is preferably from 3 or more to 24 or less, more preferably from 3 or more to 20 or less, further preferably from 5 or more to 15 or less, still further preferably from 7 or more to 14 or less, and most preferably 8 or more to 13 or less.

Examples of commercial products that can be used as component (D-2) include, for example, Unilube DGP-700 (PPG-9 diglyceryl), Unilube DGP-700F (PPG-9 diglyceryl), and Unilube DGP-950 (PPG-14 diglyceryl) which are polyoxypropylene diglyceryl ethers manufactured by Nichiyu Chemical Co., Ltd.; SY-DP9 (PPG-9 diglyceryl), SY-DP14 (PPG-14 polyglyceryl-2 ether), SY-DP14T (PPG-14 polyglyceryl-2 ether) which are polyoxypropylene diglyceryl ethers manufactured by Sakamoto Yakuhin Kogyo Co., Ltd.; and SC-P750 (POP(9) polyglyceryl ether), SC-P1000 (POP(14) polyglyceryl ether), and SC-P1600(POP(24) polyglyceryl ether) which are polyoxypropylene polyglyceryl ethers manufactured by Sakamoto Yakuhin Kogyo Co., Ltd. They may be used alone or in combination of two or more.

The content of component (D-2) in the hair cleansing composition can be adjusted as desired. For example, it can be adjusted according to the type and content of component (B) and other factors to obtain a better efficacy. From the perspective of imparting good foamability (foam amount) and good foam quality when rinsing hair, and imparting smoothness to hair during scrubbing or rinsing, the content of component (D-2) may be, for example, 0.01 mass% or more, preferably 0.025 mass% or more, more preferably 0.05 mass% or more, further preferably 0.075 mass% or more, still further preferably 0.1 mass% or more, and still further preferably 0.2 mass% or more. Furthermore, from the above-mentioned perspective, the content of component (D-2) may be, for example, 10 mass% or less, preferably 5 mass% or less, more preferably 4 mass% or less, further preferably 3 mass% or less, still further preferably 2 mass% or less, still further preferably 1 mass% or less, still further preferably 0.75 mass% or less, and still further preferably 0.6 mass% or less.

More particularly, from the above-mentioned perspective, the content of component (D-2) may be, for example, from 0.01 mass% or more to 10 mass% or less, preferably from 0.025 mass% or more to 5 mass% or less, more preferably from 0.05 mass% or more to 4 mass% or less, further preferably from 0.075 mass% or more to 3 mass% or less, still further preferably from 0.1 mass% or more to 2 mass% or less, still further preferably from 0.2 mass% or more to 1 mass% or less, still further preferably from 0.2 mass% or more to 0.75 mass% or less, and still further preferably from 0.2 mass% or more to 0.6 mass% or less.

### <Aqueous medium>

The hair cleansing composition of the present invention comprises an aqueous medium. Examples of the aqueous medium include: water; lower alcohols, such as ethanol and isopropyl alcohol; low-molecular-weight diols or triols having a carbon number of 6 or less, such as 1,3-butanediol, glycerol, ethylene glycol, 1,2-propanediol; and a mixture thereof, among which water is preferable. As the water, tap water, deionized water, or distilled water, etc. can be used. The content of the aqueous medium in the hair cleansing composition is preferably 50 mass% or more, and more preferably 60 mass% or more, relative to the total hair cleansing composition. From the same perspective, the content of the aqueous medium is preferably 95 mass% or less, and more preferably 92 mass% or less.

### <Additional components (E)>

In addition to the above-mentioned components (A), (B), (C), (D-1), (D-2) and the aqueous medium, the hair cleansing composition of this embodiment may further comprise additional components (E) that are commonly used in the art to which the present invention pertains.

Several additional components (E) that can be used in the hair cleansing composition of this embodiment are exemplified blow, but the additional components (E) that can be used in the present invention are not limited thereto.

### <Component (E-1)>

The hair cleansing composition may further comprise polypropylene glycol (PPG) as component (E-1). Here, component (E-1) represents a polymer of 3 or more molecules of propylene glycol.

From the perspective of imparting good foamability (foam amount) and good foam quality when rinsing hair, and imparting smoothness to hair during scrubbing or rinsing, the degree of polymerization of propylene glycol in component (E-1) is 3 or more, preferably 5 or more, further preferably 7 or more; and preferably 30 or less, more preferably 26 or less, further preferably 20 or less, and still further preferably 17 or less. From the above-mentioned perspective, the degree of polymerization of propylene glycol is preferably from 3 or more to 30 or less, more preferably from 5 or more to 26 or less, and further preferably from 6 or more to 20 or less.

Embodiments of polypropylene glycol as component (E-1) include PPG-3, PPG-7, PPG-9, PPG-17 and PPG-61. They may be used alone or in combination of two or more.

The content of component (E-1) in the hair cleansing composition can be adjusted as desired. For example, it can be adjusted according to the type and content of component (A) and other factors to obtain a better efficacy. From the perspective of imparting good foamability (foam amount) and good foam quality when rinsing hair, and imparting smoothness to hair during scrubbing or rinsing, the content of component (E-1) may be, for example, 0.01 mass% or more, preferably 0.025 mass% or more, more preferably 0.05 mass% or more, further preferably 0.075 mass% or more, and still further preferably 0.10 mass% or more. Furthermore, from the same perspective, the content of component (E-1) may be, for example, 5 mass% or less, more preferably 3 mass% or less, further preferably 2 mass% or less, and still further preferably 1 mass% or less.

More particularly, from the above-mentioned perspective, the content of component (E-1) may be, for example, from 0.01 mass% or more to 5 mass% or less, preferably from 0.025 mass% or more to 4 mass% or less, more preferably from 0.05 mass% or more to 3 mass% or less, further preferably from 0.075 mass% or more to 2 mass% or less, and still further preferably from 0.10 mass% or more to 1 mass% or less.

### <Component (E-2)>

Moreover, a pH adjusting agent (component (E-2)) may be added in the hair cleansing composition of this embodiment, to adjust the pH to a desired range. Embodiments of the pH adjusting agent include, for example, organic carboxylic acids, such as lactic acid, malic acid, citric acid, and succinic acid. Furthermore, as other pH adjusting agents, bases such as sodium hydroxide, potassium hydroxide, and ammonium chloride can be used in combination with these organic acids.

### <Component (E-3)>

In addition, in order to obtain desired effects such as improvement of the cleansing performance and improvement of the combing performance of hair after drying, etc., an oil (component (E-3) may be further contained in the hair cleansing composition of this embodiment. Embodiments of the oil include, for example, fatty acids such as lauric acid, and polysiloxanes.

### <Other components (E)>

In the hair cleansing composition of this embodiment, in addition to the above-mentioned components, components used in ordinary hair cleansing compositions can be appropriately added according to the desired effect. Examples of such optional components include, for example, anti-dandruff agents; vitamins; fungicides; anti-inflammatory agents such as glycyrrhizic acid and a salt thereof (e.g., dipotassium glycyrrhizate), glycyrrhetic acid and a salt thereof; preservatives such as benzoic acid and a salt thereof; chelating agents; moisturizers such as sorbitol and panthenol; colorants such as dyes and pigments; extracts such as eucalyptus extracts (e.g., polar solvent extracts of eucalyptus), conchiolin or a hydrolyzate thereof, proteins obtained from honey, royal jelly, or silk or hydrolyzates thereof, protein-containing extract obtained from the seeds of legumes, ginseng extracts, rice germ extracts, fucus extracts, aloe vera extracts, lotus extracts, pomegranate extracts, wild rose extracts, Dendranthema indicum extracts, licorice extracts, alpinia speciosa leaf extracts and Chlorella extracts; pearling agents such as titanium oxide; perfumes; UV absorber; antioxidants; shea butter; rose water; sunflower oil; orange oil; and eucalyptus oil, etc.

The pH value of the hair cleansing composition is described below.

In this embodiment, the pH value of the hair cleansing composition is tested at 25°C. From the perspective of improving the foamability or improving the detergency of the hair cleansing composition when rinsing hair, the pH value of the hair cleansing composition of the present invention may be, for example, 7 or less, preferably 6.5 or less, and more preferably 6 or less. Furthermore, from the same perspective, the pH value is preferably 2.5 or more, more preferably 3 or more, and more preferably 3.5 or more.

More particularly, from the above-mentioned perspective, the pH value may be, for example, from 2.5 or more to 7 or less, preferably from 3 or more to 6.5 or less, and more preferably from 3.5 or more to 6 or less.

Furthermore, the hair cleansing composition of this embodiment is preferably a liquid composition. In this case, although the viscosity of the hair cleansing composition at 5°C may vary according to the amounts and ratios of the above-mentioned components, and the structure or mechanism of the foam discharge device, the viscosity of the hair cleansing composition is preferably 1 mPa•s or more, more preferably 2 mPa•s or more, and further preferably 3 mPa•s or more from the perspective of foam quality to promote application to hair. Furthermore, from the perspective of improving the foam discharge performance during use when the hair cleansing composition is filled in a non-gas type foam discharge device, the viscosity of the hair cleansing composition is preferably 300 mPa•s or less, more preferably 250 mPa•s or less, further preferably 200 mPa•s or less, still further preferably 50 mPa•s or less.

More particularly, from the above-mentioned perspective, the viscosity of the hair cleansing composition may be, for example, from 1 mPa•s or more to 300 mPa•s or less, preferably from 2 mPa•s or more to 250 mPa•s or less, more preferably from 3 mPa•s or more to 200 mPa•s or less, and further preferably from 3 mPa•s or more to 50 mPa•s or less.

### <Preparation method of hair cleansing composition>

The preparation method of the hair cleansing composition of the present invention is not particularly limited. For example, the hair cleansing composition can be prepared by mixing one or more components selected from the group consisting of components (A) to (C), component (D-1) and component (D-2), and optionally other components. More particularly, the hair cleansing composition containing water is prepared by mixing one or more components selected from the group consisting of components (A) to (C), component (D-1) and component (D-2), and optionally other components, and dissolving or dispersing them in water.

In an embodiment of the present invention, component (A), component (B), component (C) and component (D-1) are added to water heated to about 50-100°C, stirred until uniform, and cooled, and during cooling component (D-2) and optionally other components are added, stirred until uniform, and then cooled to room temperature to obtain the hair cleansing composition.

### <Method of using the hair cleansing composition and kit comprising the hair cleansing composition>

The method of using the hair cleansing composition of the present invention is not particularly limited. For example, a kit comprising the hair cleansing composition of the present invention and a foam discharge device can be provided for use. For example, the hair cleansing composition of the present invention can be filled in the foam discharge device for use, or the hair cleansing composition of the present invention can be provided in the form of a supplementary pack together with the foam discharge device for use.

The foam discharge device is preferably a filled non-gas type foam discharge device for use. The non-gas type foam discharge device can be any non-gas type foam discharge device, as long as it can discharge foam after the air cleansing composition is mixed with air. For example, the non-gas type foam discharge device can be a pump-type foam-forming device or a squeeze-type foam-forming device. In the pump-type foam-forming device, a cylinder of an air chamber and a cylinder of a liquid cleansing composition are pressurized by pressing a pump head, causing the air and the liquid cleansing composition to be mixed and discharged in the form of foam. In the squeeze-type foam-forming device, a housing portion of the device (container) that is deformable is pressed to cause the container to deform so as to discharge foam. Preferably, a porous membrane filter with meshes or the like is provided in a discharge passage of the hair cleansing composition of the devices.

Embodiments of the non-gas type foam discharge device include, for example, a foam forming device manufactured by Yoshino Kogyosho Co. Ltd. and Daiwa Seikan Co. Ltd. In addition, the foam discharge devices described in Japanese Patent Publication No. 7-315463, Japanese Patent Publication No. 8-230961, and Japanese Patent Publication No. 2005-193972 can also be used.

The hair cleansing method using the hair cleansing composition of the present invention may include, for example, the steps of applying the hair cleansing composition to hair and rinsing. Furthermore, the hair cleansing method using the hair cleansing composition of the present invention preferably includes the steps of discharging the hair cleansing composition from the foam discharge device, applying it to hair, and rinsing. More preferably, the method includes applying the hair cleansing composition discharged from the foam discharge device to hair, causing the applied hair cleansing composition to foam, and rinsing. By making the hair cleansing composition into a foam and applying it to hair, the hair cleansing composition can be easily dispersed over the entire hair and thus easily spread on hair during application.

By combining specific components (A) to (C) and one or more components selected from the group consisting of components (D-1) and components (D-2), the hair cleansing composition of the present invention has excellent foam discharge performance at room temperature (such as 30°C) with which foam of good quality can be discharged; has a relatively low viscosity at a low temperature (5°C) to maintain good foam discharge performance; and imparts sufficient smoothness to hair during scrubbing or rinsing.

More specifically, according to the present invention, a hair cleansing composition having excellent foam discharge performance even in a low-temperature environment at about 5°C (e.g., a bathroom in a cold area in winter) can be obtained. Furthermore, it is possible to obtain a hair cleansing composition with excellent appearance of the discharged foam, for example, fine and uniform foam.

Moreover, the hair cleansing composition of the present invention can not only ensure the above-mentioned good foam quality and foam discharge performance, but also has good foamability when the foam is discharged from a device to apply to hair, and provides sufficient smoothness when scrubbing or rinsing hair. Therefore, for example, a user can get a good clean feeling when rinsing hair, and can also feel an excellent sebum and dirt cleansing effect and good hair smoothness.

### Examples:

### pH test

Using a pH meter (e.g., Mettler Toledo^{™} FiveEasy Plus^{™} FEP20 pH Meter, manufactured by Thermo Fisher Scientific, Inc.), the pH of a hair cleansing agent obtained from the hair cleansing composition was tested at 25°C.

### Viscosity test

Using a viscometer (e.g., BROOKFIELD DV-E Viscometer, manufactured by Brookfield Co., Ltd.), the viscosity of the hair cleansing composition was tested using rotor No. 1 at a rotational speed of 60 r.p.m at 5°C and 30°C, respectively.

### Evaluation of foam quality

About 200 ml of the hair cleansing composition was filled in a pump-type foam forming device (manufactured by Yoshino Kogyosho Co. Ltd., where filters of porous membrane mesh are provided in a foam discharge passage, with a mesh having a mesh number of #200, and a mesh having a mesh number of #305; liquid discharge volume upon one press: about 1 g, air discharge amount upon one press (foam discharge amount): about 13 cm³, device volume: about 300 ml), and allowed to stand for 4 hrs in a constant-temperature control room at 5°C and 30°C respectively. Then, the device was placed on a horizontal platform, and a pump head was vertically pressed to the bottom to discharge foam. At this time, the foam discharge performance was evaluated and classified into the following five grades:
5: very good
4: good
3: normal
2: poor
1: very poor

The same environmental conditions as above were used, the pump head was pressed 4 times instead, and the quality of the obtained foam was evaluated and divided into the following five grades:
5: very good
4: good
3: normal
2: poor
1: very poor

### Evaluation of hair smoothness during scrubbing and rinsing

A bundle of hair of 45 cm in length, 2.5 cm in width, and 20 g in weight was used as a hair bundle for evaluation. After the hair bundle for evaluation was slightly rinsed with warm water at 40°C, the hair cleansing agent obtained after two presses was applied to clean it. The smoothness of the hair bundle during scrubbing was evaluated and classified into the following five grades:
5: very good
4: good
3: normal
2: poor
1: very poor

Afterwards, the hair bundle with foam was rinsed with warm water at 40°C and a flow rate of 2 L/min. The smoothness of the hair bundle was evaluated during rinsing and divided into the following 5 grades:
5: very good
4: good
3: normal
2: poor
1: very poor

### (Examples 1-11, and Comparative Examples 1-6)

The hair cleansing compositions of various examples and comparative examples were prepared according to the formulation shown in Tables 1-3, and evaluated based on the above-mentioned methods.

**[Table 1]**

| | | Component | Example 1 | Example 2 | Example 3 | Example 4 | Example 5 | Example 6 |
|---|---|---|---|---|---|---|---|---|
| (A) Surfactants | Anionic | Ammonium laureth sulfate* 1 | 7.00 | 7.00 | 7.00 | 7.00 | 7.00 | 7.00 |
| | | Sodium laureth sulfate*2 | 3.00 | 3.00 | 3.00 | 3.00 | 3.00 | 3.00 |
| | Amphoteric | Lauryl hydroxysultaine*3 | 0.60 | 0.60 | 0.60 | 0.60 | 0.60 | 0.60 |
| | | Laurylamidopropryl betaine*4 | 0.30 | 0.30 | 0.30 | 0.30 | 0.30 | 0.30 |
| | Nonionic | Isodecyl glyceryl ether*5 | 0.70 | 0.70 | 0.70 | 0.70 | 0.70 | 0.70 |
| | | Laureth-3*6 | 0.20 | 0.20 | 0.20 | 0.20 | 0.20 | 0.20 |
| | | Laureth-16*7 | 1.80 | 1.80 | 1.80 | 1.80 | 1.80 | 1.80 |
| | | Cocoamide methyl MEA*8 | 0.45 | 0.45 | 0.45 | 0.45 | 0.45 | 0.45 |
| Total amount of (A) surfactants | | | 14.05 | 14.05 | 14.05 | 14.05 | 14.05 | 14.05 |
| Anionic surfactant/(A) | | | 0.71 | 0.71 | 0.71 | 0.71 | 0.71 | 0.71 |
| (B) Cationic polymer | | Polyquaternium-10*9 | 0.04 | 0.05 | 0.04 | 0.04 | 0.04 | 0.04 |
| (C) Dialkyl ether | | Dioctyl ether*10 | 0.50 | 0.50 | 0.50 | 0.50 | 0.50 | 0.30 |
| | | Dihexyl ether*11 | -- | -- | -- | -- | -- | -- |
| | | Didecyl ether*12 | -- | -- | -- | -- | -- | -- |
| (D-1) | | PPG-3 octyl ether* 13 | -- | -- | 1.00 | 0.40 | 0.40 | 0.40 |
| (D-2) | | PPG-9 diglyceryl ether* 14 | 0.50 | 0.50 | 0.50 | -- | 0.50 | 0.50 |
| (E) Additional components | | PPG-7 polypropylene glycol-7*15 | 0.50 | 0.50 | 0.50 | 0.50 | 0.50 | 0.50 |
| | | Malic acid*16 | Suitable amount | Suitable amount | Suitable amount | Suitable amount | Suitable amount | Suitable amount |
| | | Water | Balance | Balance | Balance | Balance | Balance | Balance |
| | | PPG-3 benzyl ether myristate*17 | -- | -- | -- | -- | -- | -- |
| | | Dioctyl carbonate *18 | -- | -- | -- | -- | -- | -- |
| Total | | | 100 | 100 | 100 | 100 | 100 | 100 |
| (C)/(B) | | | 12.5 | 10 | 12.5 | 12.5 | 12.5 | 7.5 |
| pH | | | 5.0 | 5.0 | 5.0 | 5.0 | 5.0 | 5.0 |
| Hair smoothness during scrubbing | | | 4 | 5 | 4 | 3 | 4 | 3 |
| Hair smoothness during rinsing | | | 3 | 3 | 5 | 4 | 4 | 3 |
| Viscosity/30°C | | | 3.0 | 4.8 | 8.2 | 6.8 | 5.0 | 7.2 |
| Foam discharge performance/30°C | | | 5 | 5 | 5 | 5 | 5 | 5 |
| Foam quality/30°C | | | 5 | 5 | 5 | 5 | 5 | 5 |
| Viscosity/5°C | | | 6.3 | 9.6 | 19.4 | 17.6 | 10.5 | 23.1 |
| Foam discharge performance/5°C | | | 5 | 4 | 4 | 4 | 4 | 4 |
| Foam quality/5°C | | | 5 | 5 | 4 | 4 | 5 | 4 |

| | | | | | | | | |
|---|---|---|---|---|---|---|---|---|
| *1: manufactured by Kao Co. Ltd., EMAL 125A (having 25% of an active ingredient) *2: manufactured by Kao Co. Ltd., EMAL 227-PH11 (TW) (having 27.5% of an active ingredient) *3: manufactured by Kao Co. Ltd., Amphitol 20HD (having 30% of an active ingredient) *4: manufactured by Kao Co. Ltd., Amphitol 20AB (having 30% of an active ingredient) *5: manufactured by Kao Co. Ltd., PENETOL GE-ID (having 90% of an active ingredient) *6: manufactured by Kao Co. Ltd., EMULGEN 103KG *7: manufactured by Sino-Japan Chemical Co., Ltd., SINOPOL 1116 *8: manufactured by Kao Co. Ltd., AMINON C-11S *9: manufactured by Kao Co. Ltd., CATICELO M-80 *10: BASF Ltd., CETIOL OE DEO (having 99.97% of an active ingredient) *11: Sigma-Aldrich Corporation, DIHEXYL ETHER (having 97% of an active ingredient) * 12: Tokyo Chemical Industry Co., Ltd., DIDECYL ETHER (having >95% of an active ingredient) *13: manufactured by Kao Co. Ltd., KAO SOFCARE GP-1 *14: Sakamoto Yakuhin Kogyo Co., Ltd., SY-DP9 *15: Adeka Fine Chemical Corporation, ADEKA CARPOL DL-30 (having 99.95% of an active ingredient) * 16: Fuso Chemical Co., Ltd., LIQUID MALIC ACID (having 50% of an active ingredient) *17: Croda Co., Ltd., CRODAMOL STS *18: Cognis Co., Ltd., CETIOL CC | | | | | | | | |

**[Table 2]**

| | | Component | Example 7 | Example 8 | Example 9 | Example 10 | Example 11 |
|---|---|---|---|---|---|---|---|
| (A) Surfactants | Anionic | Ammonium laureth sulfate | 7.00 | 7.00 | 7.00 | 7.00 | 7.00 |
| | | Sodium laureth sulfate | 3.00 | 3.00 | 3.00 | 3.00 | 3.00 |
| | Amphoteric | Lauryl hydroxysultaine | 0.60 | 0.60 | 0.60 | 0.60 | 0.60 |
| | | Laurylamidopropryl betaine | 0.30 | 0.30 | 0.30 | 0.30 | 0.30 |
| | Nonionic | Isodecyl glyceryl ether | 0.70 | 0.70 | 0.70 | 0.70 | 0.70 |
| | | Laureth-3 | 0.20 | 0.20 | 0.20 | 0.20 | 0.20 |
| | | Laureth-16 | 1.80 | 1.80 | 1.80 | 1.80 | 1.80 |
| | | Cocoamide methyl MEA | 0.45 | 0.45 | 0.45 | 0.45 | 0.45 |
| Total amount of (A) surfactants | | | 14.05 | 14.05 | 14.05 | 14.05 | 14.05 |
| Anionic surfactant/(A) | | | 0.71 | 0.71 | 0.71 | 0.71 | 0.71 |
| (B) Cationic polymer | | Polyquaternium-10 | 0.04 | 0.10 | 0.04 | 0.04 | 0.04 |
| (C) Dialkyl ether | | Dioctyl ether | 0.80 | 0.80 | -- | -- | 0.50 |
| | | Dihexyl ether | -- | -- | 0.80 | -- | -- |
| | | Didecyl ether | -- | -- | -- | 0.50 | -- |
| (D-1) | | PPG-3 octyl ether | 0.40 | 0.40 | 0.40 | 1.00 | 1.00 |
| (D-2) | | PPG-9 diglyceryl ether | 0.50 | 0.50 | 0.50 | 0.50 | -- |
| (E) Additional components | | PPG-7 polypropylene glycol-7 | 0.50 | 0.50 | 0.50 | 0.50 | 0.50 |
| | | Malic acid | Suitable amount | Suitable amount | Suitable amount | Suitable amount | Suitable amount |
| | | Water | Balance | Balance | Balance | Balance | Balance |
| | | PPG-3 benzyl ether myristate | -- | -- | -- | -- | -- |
| | | Dioctyl carbonate | -- | -- | -- | -- | -- |
| Total | | | 100 | 100 | 100 | 100 | 100 |
| (C)/(B) | | | 20 | 8 | 20 | 12.5 | 12.5 |
| pH | | | 5 | 5.0 | 5.0 | 5.0 | 5.0 |
| Hair smoothness during scrubbing | | | 4 | 5 | 3 | 4 | 4 |
| Hair smoothness during rinsing | | | 4 | 5 | 3 | 5 | 4 |
| Viscosity/30°C | | | 4.3 | 7.1 | 8.7 | 6.8 | 9.2 |
| Foam discharge performance/30°C | | | 5 | 4 | 5 | 5 | 5 |
| Foam quality/30°C | | | 5 | 5 | 5 | 5 | 5 |
| Viscosity/5°C | | | 7.8 | 14.1 | 33.6 | 16.6 | 28.8 |
| Foam discharge performance/5°C | | | 5 | 3 | 3 | 4 | 3 |
| Foam quality/5°C | | | 5 | 4 | 3 | 4 | 3 |

**[Table 3]**

| | | Component | Comparative Example 1 | Comparative Example 2 | Comparative Example 3 | Comparative Example 4 | Comparative Example 5 | Comparative Example 6 |
|---|---|---|---|---|---|---|---|---|
| (A) Surfactants | Anionic | Ammonium laureth sulfate | 7.00 | 7.00 | 7.00 | 7.00 | 7.00 | 7.00 |
| | | Sodium laureth sulfate | 3.00 | 3.00 | 3.00 | 3.00 | 3.00 | 3.00 |
| | Amphoteric | Lauryl hydroxysultaine | 0.60 | 0.60 | 0.60 | 0.60 | 0.60 | 0.60 |
| | | Laurylamidopropryl betaine | 0.30 | 0.30 | 0.30 | 0.30 | 0.30 | 0.30 |
| | Nonionic | Isodecyl glyceryl ether | 0.70 | 0.70 | 0.70 | 0.70 | 0.70 | 0.70 |
| | | Laureth-3 | 0.20 | 0.20 | 0.20 | 0.20 | 0.20 | 0.20 |
| | | Laureth-16 | 1.80 | 1.80 | 1.80 | 1.80 | 1.80 | 1.80 |
| | | Cocoamide methyl MEA | 0.45 | 0.45 | 0.45 | 0.45 | 0.45 | 0.45 |
| Total amount of (A) surfactants | | | 14.05 | 14.05 | 14.05 | 14.05 | 14.05 | 14.05 |
| Anionic surfactant/(A) | | | 0.71 | 0.71 | 0.71 | 0.71 | 0.71 | 0.71 |
| (B) Cationic polymer | | Polyquaternium-10 | 0.04 | 0.04 | 0.04 | 0.04 | 0.04 | 0.1 |
| (C) Dialkyl ether | | Dioctyl ether | -- | -- | 0.5 | -- | -- | 0.15 |
| | | Dihexyl ether | -- | -- | -- | -- | -- | -- |
| | | Didecyl ether | -- | -- | -- | -- | -- | -- |
| (D-1) | | PPG-3 octyl ether | -- | 1.00 | -- | 0.40 | 0.40 | 0.40 |
| (D-2) | | PPG-9 diglyceryl ether | 0.50 | 0.50 | -- | 0.50 | 0.50 | 0.50 |
| (E) Additional components | | PPG-7 polypropylene glycol-7 | 0.50 | 0.50 | 0.50 | 0.50 | 0.50 | 0.50 |
| | | Malic acid | Suitable amount | Suitable amount | Suitable amount | Suitable amount | Suitable amount | Suitable amount |
| | | Water | Balance | Balance | Balance | Balance | Balance | Balance |
| | | PPG-3 benzyl ether myristate | -- | -- | -- | 0.50 | -- | -- |
| | | Dioctyl carbonate | -- | -- | -- | -- | 0.50 | -- |
| Total | | | 100 | 100 | 100 | 100 | 100 | 100 |
| (C)/(B) | | | -- | - | 12.5 | 12.5 | 12.5 | 1.5 |
| pH | | | 5.0 | 5.0 | 5.0 | 5.0 | 5.0 | 5.0 |
| Hair smoothness during scrubbing | | | 3 | 3 | 3 | 3 | 3 | 4 |
| Hair smoothness during rinsing | | | 2 | 4 | 2 | 2 | 2 | 5 |
| Viscosity/30°C | | | 6.5 | 14.4 | 4.3 | 9.9 | 7.7 | 12.0 |
| Foam discharge performance/30°C | | | 5 | 3 | 5 | 5 | 5 | 4 |
| Foam quality/30°C | | | 5 | 4 | 5 | 5 | 5 | 4 |
| Viscosity/5°C | | | 23.0 | 76.7 | 10.9 | 54.3 | 19.6 | 49.0 |
| Foam discharge performance/5°C | | | 2 | 1 | 4 | 1 | 3 | 1 |
| Foam quality/5°C | | | 2 | 1 | 5 | 1 | 3 | 1 |

According to the above table, it can be known that compared with the comparative examples, by adding a dialkyl ether, and one or more compounds selected from the group consisting of component (D-1) and component (D-2) in the examples of the present invention, sufficient smoothness can be imparted to hair when scrubbing or rinsing, a lower viscosity is shown at low temperature, and the foam discharge performance and foam quality at low temperatures are superior.

For example, it can be seen from comparison of Example 1 of the present invention with Comparative Example 1 that the hair cleansing composition of the present invention with a dialkyl ether has a lower viscosity at 5°C, obviously better foam discharge performance and foam quality, and better hair smoothness during scrubbing or rinsing.

From the results of comparison of Example 3 of the present invention with Comparative Example 2, and Example 5 of the present invention with Comparative Examples 4 and 5, it can be known that the hair cleansing composition of the present invention with a dialkyl ether has a lower viscosity at 5°C, obviously better foam discharge performance and foam quality, and better hair smoothness t during rinsing.

In addition, it can be known from the results of comparison of Examples 1, 5, and 11 of the present invention with Comparative Example 3 that the hair cleansing composition of the present invention with one or more compounds selected from the group consisting of component (D-1) and component (D-2) has significantly better hair smoothness during scrubbing or rinsing.

Other examples of the present invention also exhibit the above effects.

### [Industrial Applicability]

The hair cleansing composition of the present invention can be preferably used in the fields of hair shampoo and the like. In addition to being suitable for use with humans, it is also suitable for use with dogs, cats and other animals.

## Claims

1. A hair cleansing composition, comprising component (A), component (B), component (C), and one or more components selected from the group consisting of component (D-1) and component (D-2), wherein:
component (A) is a surfactant comprising an anionic surfactant;
component (B) is a cationic polymer;
component (C) is a dialkyl ether represented by the general formula (4):
R⁴-O-R⁵ (4)
where in the general formula (4), R⁴ and R⁵ may be the same or different, and are each independently a linear or branched alkyl group having a carbon number from 5 or more to 14 or less;
component (D-1) is a compound represented by the general formula (5):
R^{a}-O-(AlkO)ₘ-R^{b} (5)
where in the general formula (5), R^{a} represents a linear or branched alkyl or alkenyl group having a carbon number from 8 or more to 10 or less, AlkO represents a linear or branched oxyalkylene group having a carbon number from 2 or more to 4 or less, m is a number of oxyalkylene groups and represents an average from 0.5 or more to 3.5 or less, and R^{b} represents a hydrogen atom or methyl; and
component (D-2) is a polyoxypropylene polyglyceryl ether,
wherein the mass ratio of the content of component (C) to the content of component (B) ((C)/(B)) in the hair cleansing composition is from 2 or more to 50 or less.

2. The hair cleansing composition according to claim 1, further comprising (E-1) polypropylene glycol.

3. The hair cleansing composition according to claim 1 or 2, wherein the content of component (A) in the hair cleansing composition is from 5 mass% or more to 25 mass% or less relative to the total hair cleansing composition.

4. The hair cleansing composition according to any one of claims 1 to 3, wherein component (A) further comprises one or more surfactants selected from the group consisting of a cationic surfactant, a nonionic surfactant and an amphoteric surfactant.

5. The hair cleansing composition according to any one of claims 1 to 4, wherein the content of component (B) in hair cleansing composition is from 0.01 mass% or more to 1 mass% or less relative to the total hair cleansing composition.

6. The hair cleansing composition according to any one of claims 1 to 5, wherein component (B) comprises one or more cationic polymers selected from the group consisting of cationic guar gum, PPG-2 hydroxypropyltrimethylammonium cellulose, cationic galactomannan, cationic starch, polyquaternium-6, polyquaternium-7, polyquaternium-10, polyquaternium-22, and polyquaternium-52.

7. The hair cleansing composition according to any one of claims 1 to 6, wherein the content of component (C) in the hair cleansing composition is from 0.01 mass% or more to 5 mass% or less relative to the total hair cleansing composition.

8. The hair cleansing composition according to any one of claims 1 to 7, wherein component (C) is a compound represented by the general formula (4) below:
R⁴-O-R⁵ (4)
where in the general formula (4), R⁴ and R⁵ are the same or different, and are each independently a linear or branched alkyl group having a carbon number from 3 or more to 18 or less.

9. The hair cleansing composition according to any one of claims 1 to 8, wherein the ratio of the content of the anionic surfactant to the content of component (A) ((anionic surfactant)/(A)) is from 0.25 or more to 1 or less.

10. The hair cleansing composition according to any one of claims 1 to 9, wherein component (A) further comprises a nonionic surfactant and an amphoteric surfactant.

11. A hair cleansing kit, comprising a foam discharge device, and
a hair cleansing composition according to any one of claims 1 to 10.

12. The hair cleansing kit according to claim 11, wherein the hair cleansing composition is used as a supplementary pack or filled in the foam discharge device.

13. A hair cleansing method, comprising the following steps:
discharging the hair cleansing composition according to any one of claims 1 to 10 by a foam discharge device, applying hair cleansing composition to the hair, and rinsing.

## Patentansprüche

1. Haarreinigungszusammensetzung, umfassend Komponente (A), Komponente (B), Komponente (C) und eine oder mehrere Komponenten, die ausgewählt sind aus der Gruppe bestehend aus Komponente (D-1) und Komponente (D-2), wobei:
Komponente (A) ein Tensid ist, das ein anionisches Tensid umfasst;
Komponente (B) ein kationisches Polymer ist;
Komponente (C) ein Dialkylether ist, der dargestellt ist durch die allgemeine Formel (4):
R⁴-O-R⁵ (4)
worin in der allgemeinen Formel (4) R⁴ und R⁵ gleich oder unterschiedlich sein können und jeweils unabhängig eine lineare oder verzweigte Alkylgruppe mit einer Kohlenstoffzahl von 5 oder mehr bis 14 oder weniger sind;
Komponente (D-1) eine Verbindung ist, die dargestellt ist durch die allgemeine Formel (5):
R^{a}-O- (AlkO)ₘ-R^{b} (5)
worin in der allgemeinen Formel (5) R^{a} eine lineare oder verzweigte Alkyl- oder Alkenylgruppe mit einer Kohlenstoffzahl von 8 oder mehr bis 10 oder weniger darstellt, AlkO eine lineare oder verzweigte Oxyalkylengruppe mit einer Kohlenstoffzahl von 2 oder mehr bis 4 oder weniger darstellt, m eine Anzahl von Oxyalkylengruppen ist und ein Mittel von 0,5 oder mehr bis 3,5 oder weniger darstellt, und R^{b} ein Wasserstoffatom oder Methyl darstellt; und
Komponente (D-2) ein Polyoxypropylenpolyglycerinether ist,
wobei das Massenverhältnis des Gehalts an Komponente (C) zum Gehalt an Komponente (B) ((C)/(B)) in der Haarreinigungszusammensetzung 2 oder mehr bis 50 oder weniger beträgt.

2. Haarreinigungszusammensetzung gemäß Anspruch 1, ferner umfassend (E-1) Polypropylenglykol.

3. Haarreinigungszusammensetzung gemäß Anspruch 1 oder 2, wobei der Gehalt an Komponente (A) in der Haarreinigungszusammensetzung 5 Massen-% oder mehr bis 25 Massen-% oder weniger, bezogen auf die gesamte Haarreinigungszusammensetzung, beträgt.

4. Haarreinigungszusammensetzung gemäß mindestens einem der Ansprüche 1 bis 3, wobei die Komponente (A) ferner ein oder mehrere Tenside umfasst, die ausgewählt sind aus der Gruppe bestehend aus einem kationischen Tensid, einem nichtionischen Tensid und einem amphoteren Tensid.

5. Haarreinigungszusammensetzung gemäß mindestens einem der Ansprüche 1 bis 4, wobei der Gehalt an Komponente (B) in der Haarreinigungszusammensetzung 0,01 Massen-% oder mehr bis 1 Massen-% oder weniger, bezogen auf die gesamte Haarreinigungszusammensetzung, beträgt.

6. Haarreinigungszusammensetzung gemäß mindestens einem der Ansprüche 1 bis 5, wobei die Komponente (B) ein oder mehrere kationische Polymere umfasst, die ausgewählt sind aus der Gruppe bestehend aus kationischem Guarkernmehl, PPG-2-Hydroxypropyltrimethylammoniumcellulose, kationischem Galactomannan, kationischer Stärke, Polyquaternium-6, Polyquaternium-7, Polyquaternium-10, Polyquaternium-22 und Polyquaternium-52.

7. Haarreinigungszusammensetzung gemäß mindestens einem der Ansprüche 1 bis 6, wobei der Gehalt an Komponente (C) in der Haarreinigungszusammensetzung 0,01 Massen-% oder mehr bis 5 Massen-% oder weniger, bezogen auf die gesamte Haarreinigungszusammensetzung, beträgt.

8. Haarreinigungszusammensetzung gemäß mindestens einem der Ansprüche 1 bis 7, wobei Komponente (C) eine Verbindung ist, die dargestellt ist durch die folgende allgemeine Formel (4):
R⁴-O-R⁵ (4)
worin in der allgemeinen Formel (4) R⁴ und R⁵ gleich oder verschieden sind und jeweils unabhängig eine lineare oder verzweigte Alkylgruppe mit einer Kohlenstoffzahl von 3 oder mehr bis 18 oder weniger sind.

9. Haarreinigungszusammensetzung gemäß mindestens einem der Ansprüche 1 bis 8, wobei das Verhältnis des Gehalts an anionischem Tensid zum Gehalt an Komponente (A) ((anionisches Tensid)/(A)) 0,25 oder mehr bis 1 oder weniger beträgt.

10. Haarreinigungszusammensetzung gemäß mindestens einem der Ansprüche 1 bis 9, wobei die Komponente (A) ferner ein nichtionisches Tensid und ein amphoteres Tensid umfasst.

11. Haarreinigungs-Kit, umfassend eine Schaumabgabevorrichtung und eine Haarreinigungszusammensetzung gemäß mindestens einem der Ansprüche 1 bis 10.

12. Haarreinigungs-Kit gemäß Anspruch 11, wobei die Haarreinigungszusammensetzung als Ergänzungspack verwendet oder in die Schaumabgabevorrichtung gefüllt wird.

13. Haarreinigungsverfahren, umfassend die folgenden Schritte:
Abgeben der Haarreinigungszusammensetzung gemäß mindestens einem der Ansprüche 1 bis 10 durch eine Schaumabgabevorrichtung, Auftragen der Haarreinigungszusammensetzung auf das Haar und Ausspülen.

## Revendications

1. Composition de nettoyage capillaire, comprenant un composant (A), un composant (B), un composant (C), et un ou plusieurs composants choisis dans le groupe consistant en un composant (D-1) et un composant (D-2), dans laquelle :
le composant (A) est un tensioactif comprenant un tensioactif anionique ;
le composant (B) est un polymère cationique ;
le composant (C) est un éther de dialkyle représenté par la formule générale (4) :
R⁴-O-R⁵ (4)
où, dans la formule générale (4), R⁴ et R⁵ peuvent être identiques ou différents, et sont chacun indépendamment un groupe alkyle linéaire ou ramifié présentant un nombre de carbone de 5 ou plus à 14 ou moins ;
le composant (D-1) est un composé représenté par la formule générale (5) :
R^{a}-O-(AlkO)ₘ-R^{b} (5)
où, dans la formule générale (5), R^{a} représente un groupe alkyle ou alcényle linéaire ou ramifié présentant un nombre de carbone de 8 ou plus à 10 ou moins, AlkO représente un groupe oxyalkylène linéaire ou ramifié présentant un nombre de carbone de 2 ou plus à 4 ou moins, m est un nombre de groupes oxyalkylènes et représente une moyenne de 0,5 ou plus à 3,5 ou moins, et R^{b} représente un atome d'hydrogène ou un méthyle ; et
le composant (D-2) est un éther de polyglycéryle de polyoxypropylène,
dans laquelle le rapport massique entre la teneur du composant (C) et la teneur du composant (B) ((C)/(B)) dans la composition de nettoyage capillaire est compris entre 2 ou plus et 50 ou moins.

2. Composition de nettoyage capillaire selon la revendication 1, comprenant en outre du polypropylène glycol (E-1).

3. Composition de nettoyage capillaire selon la revendication 1 ou la revendication 2, dans laquelle la teneur du composant (A) dans la composition de nettoyage capillaire est comprise entre 5 % en masse ou plus et 25 % en masse ou moins par rapport à la totalité de la composition de nettoyage capillaire.

4. Composition de nettoyage capillaire selon l'une quelconque des revendications 1 à 3, dans laquelle le composant (A) comprend en outre un ou plusieurs tensioactifs choisis dans le groupe consistant en un tensioactif cationique, un tensioactif non-ionique et un tensioactif amphotère.

5. Composition de nettoyage capillaire selon l'une quelconque des revendications 1 à 4, dans laquelle la teneur de composant (B) dans la composition de nettoyage capillaire est comprise entre 0,01 % en masse ou plus et 1 % en masse ou moins par rapport à la totalité de la composition de nettoyage capillaire.

6. Composition de nettoyage capillaire selon l'une quelconque des revendications 1 à 5, dans laquelle le composant (B) comprend un ou plusieurs polymères cationiques choisis dans le groupe consistant en gomme de guar cationique, PPG-2 hydroxypropyltriméthylammonium cellulose, galactomannane cationique, amidon cationique, polyquaternium-6, polyquaternium-7, polyquaternium-10, polyquaternium-22 et polyquaternium-52.

7. Composition de nettoyage capillaire selon l'une quelconque des revendications 1 à 6, dans laquelle la teneur de composant (C) dans la composition de nettoyage capillaire est comprise entre 0,01 % en masse ou plus et 5 % en masse ou moins par rapport à la totalité de la composition de nettoyage capillaire.

8. Composition de nettoyage capillaire selon l'une quelconque des revendications 1 à 7, dans laquelle le composant (C) est un composé représenté par la formule générale (4) ci-dessous :
R⁴-O-R⁵ (4)
où, dans la formule générale (4), R⁴ et R⁵ sont identiques ou différents, et sont chacun indépendamment un groupe alkyle linéaire ou ramifié présentant un nombre de carbone de 3 ou plus et de 18 ou moins.

9. Composition de nettoyage capillaire selon l'une quelconque des revendications 1 à 8, dans laquelle le rapport entre la teneur du tensioactif anionique et la teneur du composant (A) ((tensioactif anionique/(A)) est de 0,25 ou plus et 1 ou moins.

10. Composition de nettoyage capillaire selon l'une quelconque des revendications 1 à 9, dans laquelle le composant (A) comprend en outre un tensioactif non-ionique et un tensioactif amphotère.

11. Kit de nettoyage capillaire, comprenant un dispositif distributeur de mousse, et
une composition de nettoyage capillaire selon l'une quelconque des revendications 1 à 10.

12. Kit de nettoyage capillaire selon la revendication 11, dans lequel la composition de nettoyage capillaire est utilisée en tant qu'un ensemble supplémentaire ou introduite dans le dispositif distributeur de mousse.

13. Procédé de nettoyage capillaire, comprenant les étapes suivantes :
une distribution de la composition de nettoyage capillaire selon l'une quelconque des revendications 1 à 10 par un dispositif distributeur de mousse, une application de la composition de nettoyage capillaire sur les cheveux, puis un rinçage.
